# EUROPEAN PATENT SPECIFICATION

(11) **EP 4 119 999 B1**
(45) Date of publication and mention of the grant of the patent: **29.04.2026**
(21) Application number: 21767423.3
(22) Date of filing: 04.02.2021
(51) Int. Cl.: G02B 23/24, G02B 23/26, A61B 1/00, A61B 1/05, A61B 1/06, A61B 1/07

(54) **ENDOSCOPE SYSTEM AND CONTROL PROGRAM**
ENDOSKOPSYSTEM UND STEUERUNGSPROGRAMM
SYSTÈME D'ENDOSCOPE ET PROGRAMME DE COMMANDE

(30) Priority: 11.03.2020 JP 2020042148
(43) Date of publication of application: 18.01.2023
(73) Proprietor: FUJIFILM Corporation, Tokyo 106-8620 (JP)
(72) Inventor: SHIMOMURA, Koji, Ashigarakami-gun, Kanagawa 258-8538 (JP)
(74) Representative: Dehns Germany Partnerschaft mbB
(86) International application number: PCT/JP2021/004175
(87) International publication number: WO 2021/181967

(56) References cited:
- EP-A1- 2 526 854
- WO-A1-2016/189629
- WO-A1-2019/016912
- JP-A- 2012 152 279
- JP-A- 2012 239 815
- JP-A- 2012 239 816
- JP-A- 2019 042 156
- US-A1- 2014 049 626
- US-A1- 2019 069 769

## Description

### BACKGROUND OF THE INVENTION

### 1. Field of the Invention

The present invention relates to an endoscope system and a control program.

### 2. Description of the Related Art

In the related art, an endoscope system is known in which continuous imaging is performed while irradiating a subject with normal light, such as white light, to display a live image. In addition, there is known an endoscope system that performs continuous imaging while irradiating a subject with special light, such as narrow band light, and performs analysis such as image-enhanced endoscopy (IEE).

JP2015-8782A discloses an endoscope apparatus that detects an uneven portion adaptively in accordance with an observation state by increasing a size of the extracted uneven portion in a case in which distance information is large (that is, screening observation) and reducing the size of the extracted uneven portion in a case in which the distance information is small (that is, magnified observation).

WO2018/159461A discloses an endoscope system that, based on an imaging size signal, executes determination processing of obtaining information that further contributes to diagnosis based on a characteristic of a specific part or the like in a case in which an observation target is observed in a magnified manner and skips the determination processing in a case in which the observation target is not observed in a magnified manner.

### SUMMARY OF THE INVENTION

However, in the related art, it is not possible to present a highly accurate result of the analysis in accordance with an observation situation. For example, an appropriate characteristic of illumination light used for imaging for the analysis may vary in accordance with a type of the analysis. Moreover, in a case in which the illumination light having an inappropriate characteristic is used in the analysis to be executed, a highly accurate result cannot be obtained in the analysis.

In addition, in the configurations of JP2015-8782A and WO2018/159461A, a plurality of types of analysis having different characteristics of appropriate illumination light are not switched and executed, and the idea regarding the characteristic of the appropriate illumination light in accordance with the type of the analysis is not disclosed in JP2015-8782A and WO2018/159461A. US 2019/069769 A1 discloses the preamble of claim 1.

The present invention has been made in view of the above circumstances, and is to provide an endoscope system and a control program that can present a highly accurate result of analysis in accordance with an observation situation.

An aspect of the present invention relates to an endoscope system according to claim 1. The endoscope system comprises a light source that is able to switch between a plurality of types of illumination light having different characteristics to perform irradiation, a display that is able to switch between results of a plurality of types of analysis based on a captured image obtained by imaging using the illumination light to display the results, and a processor that switches analysis of which a result is displayed on the display among the plurality of types of analysis and the characteristic of the illumination light emitted by the light source in accordance with a result of determining a size of a target area of the imaging inside a subject.

In addition, still another aspect of the present invention relates to a control program of an endoscope system according to claim 15. The control program includes a light source that is able to switch between a plurality of types of illumination light having different characteristics to perform irradiation, and a display that is able to switch between results of a plurality of types of analysis based on a captured image obtained by imaging using the illumination light to display the results, the program causing a computer to execute a process comprising switching analysis of which a result is displayed on the display among the plurality of types of analysis and the characteristic of the illumination light emitted by the light source in accordance with a result of determining a size of a target area of the imaging inside a subject.

According to the present invention, it is possible to provide the endoscope system and the control program that can present a highly accurate result of analysis in accordance with an observation situation.

### BRIEF DESCRIPTION OF THE DRAWINGS

Fig. 1 is a view showing an example of an endoscope apparatus 100 which is one embodiment of the present invention.
Fig. 2 is a schematic view showing an internal configuration of the endoscope apparatus 100 shown in Fig. 1.
Fig. 3 is a diagram showing an example of a spectrum of light generated by a light source device 5 shown in Fig. 2.
Fig. 4 is a schematic plan view showing a schematic configuration of an imaging element 23 shown in Fig. 2.
Fig. 5 is a diagram showing an example of functional blocks of a system control unit 44 of a signal processing unit 42 shown in Fig. 2.
Fig. 6 is a diagram showing an example of a screen displayed on a display 7 during a wide-area observation.
Fig. 7 is a diagram showing an example of the screen displayed on the display 7 during a detailed observation.
Fig. 8 is a diagram showing an example of switching of analysis and illumination light in the endoscope apparatus 100.

### DESCRIPTION OF THE PREFERRED EMBODIMENTS

In the following, an embodiment of the present invention will be described with reference to the drawings.

### (Embodiment)

### <Endoscope Apparatus 100 Which Is One Embodiment of Present Invention>

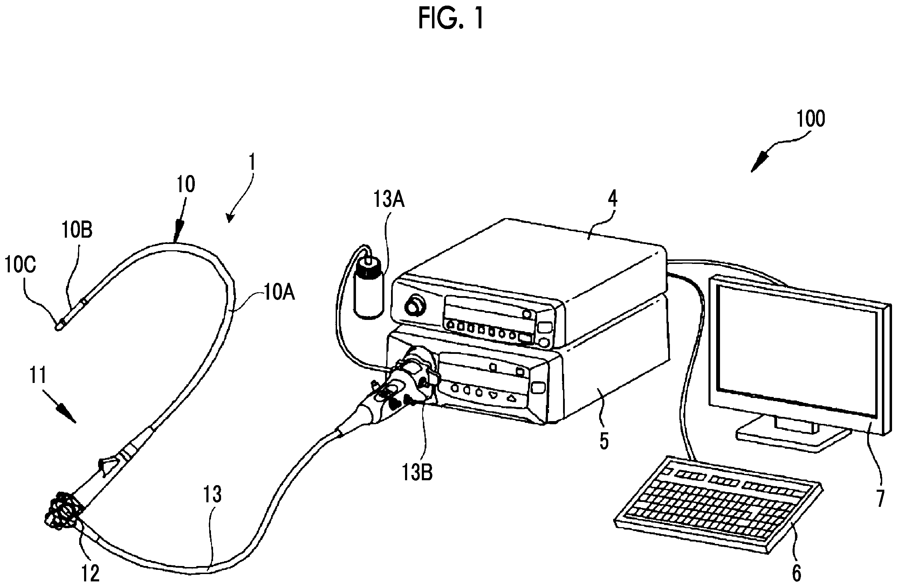

Fig. 1 is a view showing an example of an endoscope apparatus 100 which is one embodiment of the present invention.

The endoscope apparatus 100 is an example of an endoscope system according to the embodiment of the present invention. As shown in Fig. 1, the endoscope apparatus 100 comprises an endoscope 1, and a control device 4 and a light source device 5 to which the endoscope 1 is connected. The light source device 5 is an example of a light source capable of switching between a plurality of types of illumination light having different characteristics to perform irradiation.

A display 7 that displays a captured image or the like obtained by imaging an inside of a subject by the endoscope 1 and an input unit 6, which is an interface for inputting various types of information to the control device 4 are connected to the control device 4. The control device 4 controls the endoscope 1, the light source device 5, and the display 7.

The display 7 has a display surface on which display pixels are two-dimensionally arranged, and pixel data constituting image data is drawn on each display pixel on the display surface, thereby performing the display of an image based on the image data. The display 7 switches the display image in accordance with the command from the control device 4. In addition, the display 7 constitutes a display capable of switching and displaying the results of a plurality of types of analysis based on the captured image obtained by imaging.

The endoscope 1 includes an insertion part 10 which is a tubular member extending in one direction and is inserted into the subject, an operating part 11 which is provided in a base end part of the insertion part 10 and includes an operation member for performing an observation mode switching operation, an imaging recording operation, a forcep operation, an air supply/water supply operation, and a suction operation, an angle knob 12 provided adjacent to the operating part 11, and a universal cord 13 including connector portions 13A and 13B that detachably connect the endoscope 1 to the control device 4 and the light source device 5, respectively.

It should be noted that, although not shown in Fig. 1, various channels, such as a forcep hole for inserting forceps for sampling a living body tissue, such as cells or polyps, an air supply/water supply channel, and a suction channel, are provided inside the operating part 11 and the insertion part 10.

The insertion part 10 is composed of a flexible part 10A having flexibility, a bendable part 10B provided at a distal end of the flexible part 10A, and a hard distal end part 10C provided at a distal end of the bendable part 10B.

The bendable part 10B is configured to be bendable by a rotational movement operation of the angle knob 12. Depending on the site of the subject in which the endoscope 1 is used, the bendable part 10B can be bent in any direction and at any angle, and the distal end part 10C can be directed in a desired direction.

### <Internal Configuration of Endoscope Apparatus 100 Shown in Fig. 1>

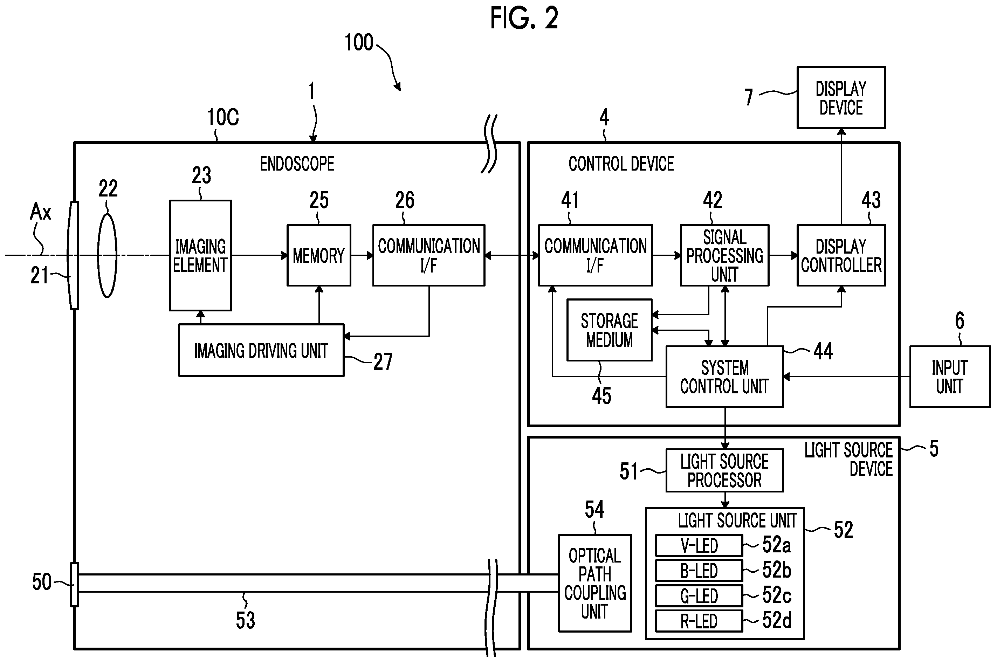

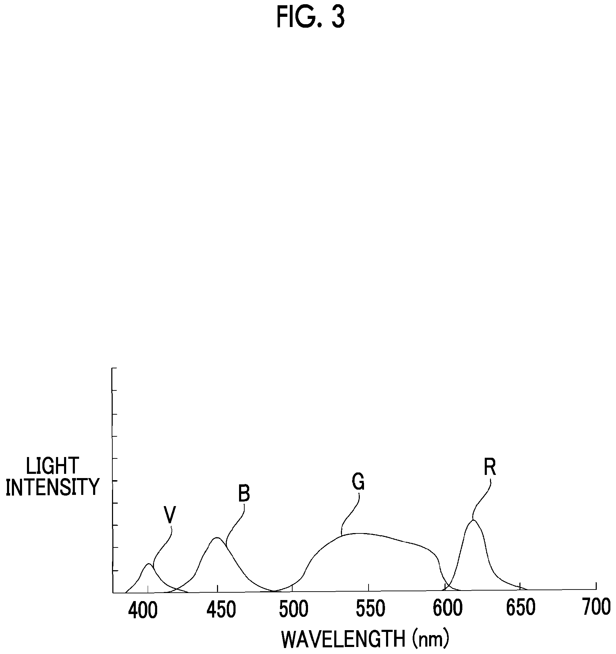

Fig. 2 is a schematic view showing an internal configuration of the endoscope apparatus 100 shown in Fig. 1. Fig. 3 is a diagram showing an example of a spectrum of light generated by the light source device 5 shown in Fig. 2.

The light source device 5 can switch between normal light and special light as illumination light and perform irradiation. The normal light is light having an emission spectrum suitable for recognition by a human, such as a doctor, such as white light. The special light is light having an emission spectrum suitable for image analysis by a computer, such as IEE, which has a different emission spectrum from the normal light.

Specifically, the light source device 5 comprises a light source processor 51, a light source unit 52, and an optical path coupling unit 54. The light source processor 51 is connected to the system control unit 44 of the control device 4, and controls the light source unit 52 based on the command from the system control unit 44.

The light source unit 52 has, for example, a plurality of semiconductor light sources, each of which is turned on or off, and in a case in which the light source unit 52 is turned on, the emission amount of each semiconductor light source is controlled to emit the illumination light for illuminating an observation target. In the present embodiment, the light source unit 52 has LEDs of four colors, a violet light emitting diode (V-LED) 52a, a blue light emitting diode (B-LED) 52b, a green light emitting diode (G-LED) 52c, and a red light emitting diode (R-LED) 52d.

By independently controlling each of the V-LED 52a, the B-LED 52b, the G-LED 52c, and the R-LED 52d, the light source processor 51 can emit violet light V, blue light B, green light G, or red light R by independently changing a light amount. As shown in Fig. 3, the V-LED 52a generates the violet light V of which a central wavelength is in a range of 405±10 nm and a wavelength range is in a range of 380 to 420 nm. The B-LED 52b generates the blue light B of which a central wavelength is in a range of 450±10 nm and a wavelength range is in a range of 420 to 500 nm. The G-LED 52c generates the green light G of which a wavelength range is in a range of 480 to 600 nm. The R-LED 52d generates the red light R of which a central wavelength is in a range of 620 to 630 nm and a wavelength range is in a range of 600 to 650 nm.

In addition, in a case of irradiation with the normal light, the light source processor 51 controls each of the LEDs 52a to 52d to emit the white light in which a light amount ratio of the violet light V, the blue light B, the green light G, and the red light R is Vc:Bc:Gc:Rc. It should be noted that Vc, Bc, Gc, Rc > 0.

In addition, in a case of irradiation with the special light, the light source processor 51 controls each of the LEDs 52a to 52d to emit the special light in which the light amount ratio of the violet light V, the blue light B, the green light G, and the red light R as short-wavelength narrow-band light is Vs:Bs:Gs:Rs.

The light amount ratio Vs:Bs:Gs:Rs is different from the light amount ratio Vc:Bc:Gc:Rc used in a case of the irradiation with the normal light, and is appropriately determined in accordance with the observation purpose. For example, in a case in which superficial blood vessels are enhanced, it is preferable to make Vs larger than Bs, Gs, and Rs, and in a case in which mesopelagic blood vessels are enhanced, it is preferable to make Gs larger than Vs, Gs, and Rs.

The optical path coupling unit 54 combines each light emitted from the V-LED 52a, the B-LED 52b, the G-LED 52c, and the R-LED 52d, and emits the combined light as the illumination light. The illumination light emitted from the optical path coupling unit 54 of the light source unit 52 enters a light guide 53 to be described below built in the universal cord 13, and is emitted to the subject through an illumination lens 50 provided at the distal end part 10C of the insertion part 10.

In the distal end part 10C of the endoscope 1, an imaging optical system including an objective lens 21 and a lens group 22, an imaging element 23 that images the subject through the imaging optical system, a memory 25, such as a random access memory (RAM), a communication interface (I/F) 26, an imaging driving unit 27, and the light guide 53 for guiding the illumination light emitted from the light source unit 52 to the illumination lens 50 are provided.

The light guide 53 extends from the distal end part 10C to the connector portion 13A of the universal cord 13. The illumination light emitted from the light source unit 52 of the light source device 5 can enter the light guide 53 in a state in which the connector portion 13A of the universal cord 13 is connected to the light source device 5.

A charge coupled device (CCD) image sensor or a complementary metal oxide semiconductor (CMOS) image sensor is used as the imaging element 23. In the present embodiment, the imaging element 23 is the CMOS using a rolling shutter.

The imaging element 23 has a light-receiving surface on which a plurality of pixels are two-dimensionally arranged, and converts an optical image formed on the light-receiving surface by the imaging optical system described above into an electrical signal (imaging signal) in each pixel. Moreover, the imaging element 23 converts the converted imaging signal from an analog signal into a digital signal having a predetermined number of bits, and outputs the imaging signal converted into the digital signal to the memory 25. For example, an imaging element on which a color filter, such as an elementary color or a complementary color, is mounted, is used as the imaging element 23. A set of the imaging signals output from the pixels of the light-receiving surface of the imaging element 23 is referred to as a captured image signal.

The imaging element 23 may be disposed at the distal end part 10C in a state in which the light-receiving surface is perpendicular to an optical axis Ax of the objective lens 21, or may be disposed at the distal end part 10C in a state in which the light-receiving surface is parallel to the optical axis Ax of the objective lens 21.

The imaging optical system provided in the endoscope 1 is composed of optical members (including the lens group 22 described above), such as a lens and a prism, which are present on an optical path of the light from the subject between the imaging element 23 and the objective lens 21, and the objective lens 21. There is also a case in which the imaging optical system is composed of only the objective lens 21. The objective lens 21 or the lens group 22 may be a lens having a variable angle of view (focal length) (for example, a zoom lens).

The memory 25 transitorily records the digital imaging signal output from the imaging element 23.

The communication I/F 26 is connected to a communication interface (I/F) 41 of the control device 4. The communication I/F 26 transmits the imaging signal recorded in the memory 25 to the control device 4 through a signal line in the universal cord 13.

The imaging driving unit 27 is connected to the system control unit 44 of the control device 4 via the communication I/F 26. The imaging driving unit 27 drives the imaging element 23 and the memory 25 based on the command from the system control unit 44 received by the communication I/F 26.

The control device 4 comprises the communication I/F 41, which is connected to the communication I/F 26 of the endoscope 1 by the universal cord 13, a signal processing unit 42, a display controller 43, the system control unit 44, and a recording medium 45.

The communication I/F 41 receives the imaging signal transmitted from the communication I/F 26 of the endoscope 1 to transmit the imaging signal to the signal processing unit 42.

The signal processing unit 42 has a memory that transitorily records the imaging signal received from the communication I/F 41 built therein, and performs processing (image processing, such as demosaicing processing or gamma correction processing) on the captured image signal that is a set of the imaging signals recorded in the memory to generate captured image information in such a format that recognition processing or the like can be performed. The captured image information generated by the signal processing unit 42 is recorded on the recording medium 45, such as a hard disk or a flash memory.

The display controller 43 displays a captured image based on the captured image information generated by the signal processing unit 42 on the display 7. A coordinate of each pixel data constituting the captured image information generated by the signal processing unit 42 is managed in association with a coordinate of any of the display pixels constituting the display surface of the display 7.

The system control unit 44 controls each unit of the control device 4, and transmits the command to the imaging driving unit 27 of the endoscope 1 and the light source processor 51 of the light source device 5, and integrally controls the entire endoscope apparatus 100. For example, the system control unit 44 performs the control of the imaging element 23 via the imaging driving unit 27. In addition, the system control unit 44 performs the control of the light source unit 52 via the light source processor 51.

The control device 4 is composed of various processors that execute a program to perform processing, a RAM, and a read only memory (ROM).

Examples of various processors include a central processing unit (CPU), which is a general-purpose processor that executes the program to perform various types of processing, a programmable logic device (PLD), which is a processor of which the circuit configuration can be changed after the manufacture, such as a field programmable gate array (FPGA), and a dedicated electric circuit, which is a processor having the circuit configuration specially designed for executing specific processing, such as an application specific integrated circuit (ASIC).

More specifically, the structure of these various processors is an electric circuit in which circuit elements, such as semiconductor elements, are combined.

The system control unit 44 or the signal processing unit 42 may be composed of one of the various processors, or may be composed of a combination (for example, a combination of a plurality of FPGAs or a combination of a CPU and an FPGA) of two or more processors of the same type or different types.

### <Schematic Configuration of Imaging Element 23 Shown in Fig. 2>

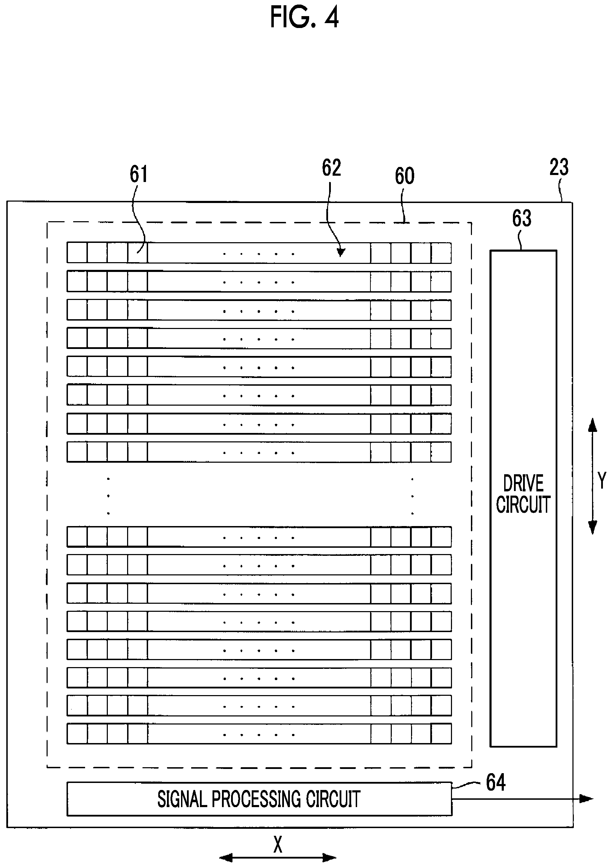

Fig. 4 is a schematic plan view showing a schematic configuration of the imaging element 23 shown in Fig. 2.

The imaging element 23 comprises an imaging surface 60 on which a plurality of pixel rows 62 consisting of a plurality of pixels 61 arranged in a row direction X are arranged in a column direction Y orthogonal to the row direction X, a drive circuit 63 that drives the pixels 61 arranged on the imaging surface 60, and a signal processing circuit 64 that processes the pixel signal read out from each pixel 61 of the pixel row 62 arranged on the imaging surface 60 into the signal line. The imaging surface 60 constitutes the light-receiving surface.

In the following, in Fig. 4, an end portion of the imaging surface 60 on one end side (upper side in Fig. 4) in the column direction Y is referred to as an upper end, and an end portion of the imaging surface 60 on the other end side (lower side in Fig. 4) in the column direction Y is referred to as a lower end.

The drive circuit 63 shown in Fig. 4 independently drives each pixel row 62 based on the signal from the imaging driving unit 27, and performs the reset of each pixel 61 included in the pixel row 62 (discharge of charge accumulated in the photoelectric conversion element), the reading out of the pixel signal in accordance with the charge accumulated in the photoelectric conversion element of each pixel 61 into the signal line, and the like.

The signal processing circuit 64 shown in Fig. 4 performs correlated double sampling processing on the pixel signal read out from each pixel 61 of the pixel row 62 into the signal line, converts the pixel signal subjected to the correlated double sampling processing into the digital signal, and outputs the converted pixel signal. The signal processing circuit 64 is controlled by the imaging driving unit 27.

The signal processing unit 42 performs the signal processing, such as the demosaicing processing and the gamma correction processing, on the pixel signal output from the imaging element 23 to generate the captured image information.

The endoscope apparatus 100 is equipped with a continuous imaging mode that continuously generates a plurality of pieces of the captured image information in accordance with one imaging instruction. In the continuous imaging mode, the system control unit 44 drives the imaging element 23 by the imaging driving unit 27 by a rolling shutter system to image the subject.

The driving of the rolling shutter system includes the rolling reset driving and the rolling read-out driving. The rolling reset driving is driving in which processing of resetting each pixel 61 of the pixel row 62 and starting the exposure of each pixel 61 is sequentially performed while changing the pixel row 62. The rolling read-out driving is driving in which processing of reading out the signal from each pixel 61 of the exposed pixel row 62 and terminating the exposure of the pixel row 62 is sequentially performed while changing the pixel row 62.

### <Functional Blocks of System Control Unit 44 of Signal Processing Unit 42 Shown in Fig. 2>

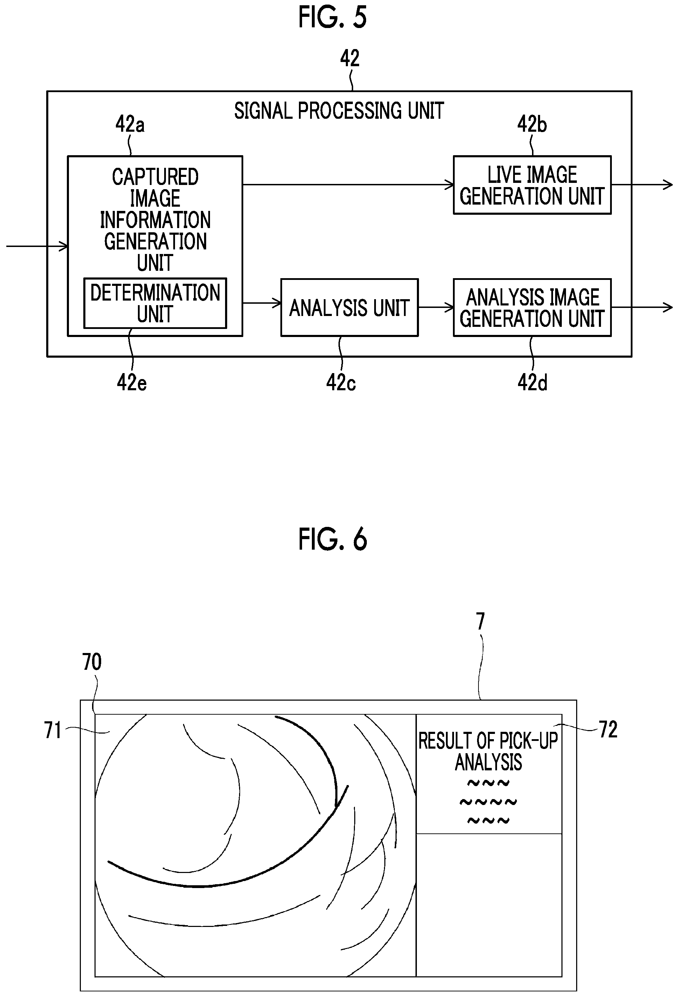

Fig. 5 is a diagram showing an example of functional blocks of the system control unit 44 of the signal processing unit 42 shown in Fig. 2.

A processor of the signal processing unit 42, for example, executes a control program stored in the ROM built in the signal processing unit 42 to function as a captured image information generation unit 42a, a live image generation unit 42b, an analysis unit 42c, and an analysis image generation unit 42d.

The captured image information generation unit 42a generates the captured image information by performing the image processing, such as the demosaicing processing or the gamma correction processing, on the imaging signal obtained by imaging of the imaging element 23. The captured image information generation unit 42a outputs the captured image information based on the imaging signal obtained by imaging during the irradiation with the normal light to the live image generation unit 42b as an imaging frame in the generated captured image information, and outputs the captured image information based on the imaging signal obtained by imaging during the irradiation with the special light to the analysis unit 42c as an imaging frame. The imaging frame is the imaging signal obtained by one imaging.

In addition, the captured image information generation unit 42a includes a determination unit 42e. The determination unit 42e determines, for example, a size of a target area of imaging by the imaging element 23 inside the subject into which the endoscope 1 is inserted, based on the captured image information generated by the captured image information generation unit 42a.

The target area of imaging by the imaging element 23 is, for example, an area inside the subject that is reflected in the captured image which is obtained by imaging and output to the live image generation unit 42b as the imaging frame. The captured image information used by the determination unit 42e for the determination may be the captured image information of the normal light, may be the captured image information of the special light, or may be the captured image information of each of the normal light and the special light.

For example, in a case in which a user (for example, a doctor) of the endoscope 1 inserts the endoscope 1 into the subject and performs an examination, first, a wide-area observation is performed for searching for an abnormal site, such as a lesion, while observing a wide area in the subject. During the wide-area observation, the user widens the target area of imaging by increasing a distance between an inner surface of the subject and the distal end (imaging element 23) of the endoscope 1, by widening the angle of view in a case in which the angle of view of the objective lens 21 or the lens group 22 is variable, or by using a digital zoom.

In addition, in a case in which the user finds the abnormal site by the wide-area observation, the user performs detailed observation of observing the abnormal site in detail. During the detailed observation, the user narrows the target area of imaging by reducing the distance between an inner surface of the subject and the distal end of the endoscope 1, by narrowing the angle of view in a case in which the angle of view of the objective lens 21 or the lens group 22 is variable, or by using the digital zoom.

The determination unit 42e determines the size of the target area of imaging, which is changed in accordance with the observation situation, and outputs the result of the determination to the analysis unit 42c. For example, the determination unit 42e determines the size of the target area of imaging in two stages of "wide" and "narrow". "Wide" constitutes a first size. "Narrow" constitutes a second size that is narrower than the first size.

The determination of the size of the target area of imaging based on the captured image information is made by, for example, using a trained model of artificial intelligence (AI) obtained by learning based on a large number of samples of a combination of the captured image and the size of the target area of imaging. Alternatively, the determination may be made based on the captured image and a predetermined algorithm. It should be noted that, as will be described below, the determination method by the determination unit 42e is not limited to these.

The live image generation unit 42b generates live image information for displaying the live image based on the imaging frame of the normal light output from the captured image information generation unit 42a, and outputs the generated live image information to the display controller 43 (see Fig. 2) as the captured image information. The live image is the motion picture that displays a result of continuous imaging by the imaging element 23 in real time.

The analysis unit 42c performs the analysis (image analysis) based on the imaging frame of the special light output from the captured image information generation unit 42a, and outputs a result of the analysis to the analysis image generation unit 42d. In addition, the analysis unit 42c can perform the plurality of types of analysis based on the imaging frame output from the captured image information generation unit 42a. The analysis unit 42c switches the analysis to be executed, based on the result of the determination of the size of the target area of imaging notified from the determination unit 42e.

For example, the plurality of types of analysis that can be executed by the analysis unit 42c include a first analysis of determining the presence or absence of the abnormal site, such as the lesion, and a second analysis of analyzing the abnormal site. The first analysis is associated with "wide" among the results of the determination by the determination unit 42e. The second analysis is associated with "narrow" among the results of the determination by the determination unit 42e. The analysis unit 42c performs the first analysis in a case in which the determination unit 42e outputs "wide" as the result of the determination, and performs the second analysis in a case in which the determination unit 42e outputs "narrow" as the result of the determination.

The first analysis is, for example, pick-up analysis of extracting the abnormal site, such as the lesion, from the captured image. The result of the pick-up analysis may be, for example, information, such as the number of extracted abnormal sites, or may be an image in which the abnormal site is highlighted in the captured image. The first analysis may be performed by using a trained model of AI obtained by learning based on a large number of samples of a combination of the captured image and the extraction result of the abnormal site, or may be performed based on the captured image and a predetermined algorithm.

The second analysis is, for example, differentiation analysis including at least any analysis of a type of the lesion or an extent of the lesion in the abnormal site. The differentiation analysis is, for example, information indicating the type of the lesion in the abnormal site of the target by a character string or the like, or information indicating the extent of the lesion in the abnormal site of the target by a numerical value or the like. The second analysis may be performed by using a trained model of AI obtained by learning based on a large number of samples of a combination of the captured image and the result of the analysis of the type or the extent of the lesion, or may be performed based on the captured image and a predetermined algorithm.

In addition, the analysis unit 42c may perform contour extraction of the captured image as the analysis in parallel with the pick-up analysis or differentiation analysis described above or by switching from these analyses. For example, the analysis unit 42c specifies the contour of a biological structure reflected in the image indicated by the captured image information obtained by imaging during the irradiation with the special light. Examples of the biological structure of a specific target include a superficial blood vessel structure, a middle layer blood vessel structure, or a deep blood vessel structure. The analysis by the analysis unit 42c is performed in parallel with displaying of the live image.

The analysis image generation unit 42d generates analysis result image information for displaying an analysis result image indicating the result of the analysis output from the analysis unit 42c, and outputs the generated analysis result image information to the display controller 43 (see Fig. 2). The analysis result image includes an image indicating the result of the pick-up analysis described above, an image indicating the result of the differentiation analysis, an IEE image indicating the result of the contour extraction, and the like.

The IEE image is an image in which the contour of the structure of the subject is enhanced based on the imaging signal obtained by imaging during the irradiation with the special light, such as a blue laser. In this case, the special light, such as the blue laser, constitutes light for image-enhanced endoscopy. For example, the IEE image is an image in which the superficial blood vessel structure is enhanced, an image in which the middle layer blood vessel structure is enhanced, an image in which the deep blood vessel structure is enhanced, and the like.

### <Screen Displayed on Display 7>

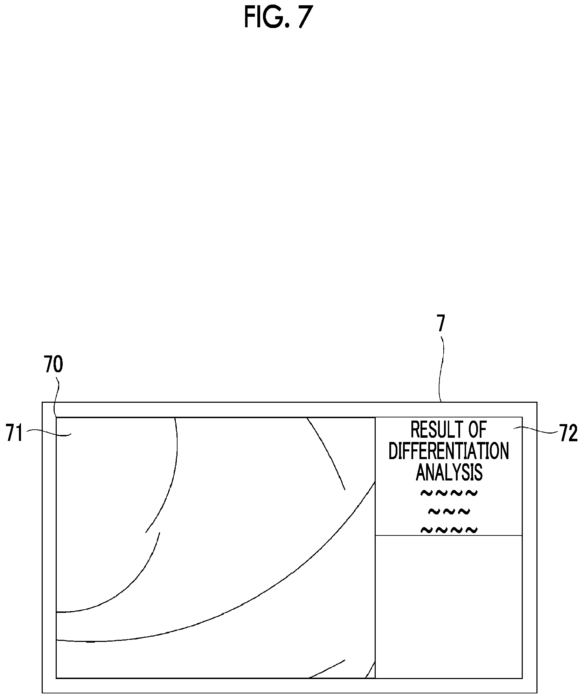

Fig. 6 is a diagram showing an example of a screen displayed on the display 7 during the wide-area observation. Fig. 7 is a diagram showing an example of the screen displayed on the display 7 during the detailed observation.

The display controller 43 displays, for example, a screen 70 shown in Figs. 6 and 7 on the display 7 based on the captured image information and the analysis result image information output from the signal processing unit 42. The screen 70 includes a main screen 71 and the sub-screen 72.

The live image based on the live image information output from the live image generation unit 42b of the signal processing unit 42 is displayed on the main screen 71. The analysis result image based on the analysis result image information output from the analysis image generation unit 42d of the signal processing unit 42 is displayed on the sub-screen 72.

As described above, the endoscope apparatus 100 displays the screen 70 including the live image based on the captured image information obtained by imaging in the first period in which the normal light is emitted, and the result of the analysis based on the captured image information obtained by imaging in the second period in which the special light is emitted.

In a case in which the user performs the wide-area observation, the determination unit 42e determines that the size of the target area of imaging is "wide", and as a result, the analysis unit 42c executes the pick-up analysis. Therefore, as shown in Fig. 6, the result of the pick-up analysis is displayed on the sub-screen 72.

In a case in which the user performs the detailed observation, the determination unit 42e determines that the size of the target area of imaging is "narrow", and as a result, the analysis unit 42c executes the differentiation analysis. Therefore, as shown in Fig. 7, the result of the differentiation analysis is displayed on the sub-screen 72.

As described above, the endoscope apparatus 100 switches the analysis of which the result is displayed on the display 7 among the plurality of types of analysis by switching the analysis to be executed, in accordance with the result of determining the size of the target area of imaging inside the subject.

It should be noted that the endoscope apparatus 100 may switch the analysis of which the result is displayed on the display 7 among the plurality of types of analysis in accordance with the result of determining the size of the target area of imaging inside the subject, while executing the plurality of types of analysis in parallel.

Although the configuration has been described in which a result of the analysis of the analysis unit 42c shown in Fig. 5 is displayed on the display 7 together with the live image, but a usage form of the result of the analysis of the analysis unit 42c is not limited to this. For example, the result of the analysis of the analysis unit 42c may be displayed by a display different from the display 7, or may be transmitted to and stored in the storage unit of the endoscope apparatus 100 or another device.

In addition, in a case in which the analysis unit 42c performs the contour extraction of the captured image in parallel with the pick-up analysis or the differentiation analysis, the display controller 43 may display the IEE image output from the signal processing unit 42 on a portion of the screen 70 different from the main screen 71 and the sub-screen 72, or a display different from the display 7.

### < Switching of Analysis and Illumination Light in Endoscope Apparatus 100>

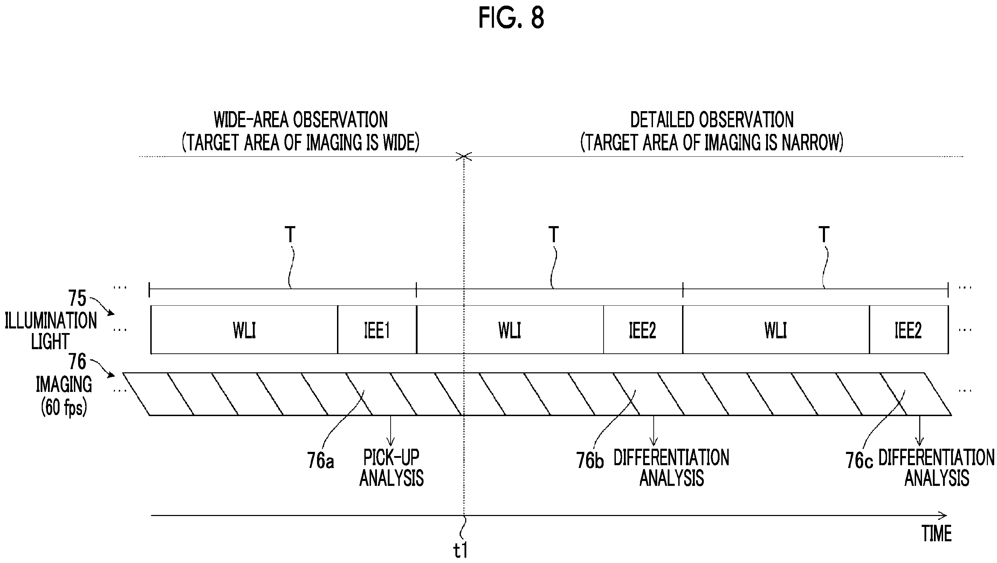

Fig. 8 is a diagram showing an example of switching of the analysis and the illumination light in the endoscope apparatus 100.

In Fig. 8, a horizontal axis represents a time. In the example shown in Fig. 8, the user of the endoscope apparatus 100 performs the wide-area observation described above until a time t1 and performs the detailed observation after a time t1.

An illumination light timing 75 is a timing at which the light source device 5 emits the illumination light in accordance with the command from the control device 4. The WLI in the illumination light timing 75 is a timing at which the light source device 5 emits the normal light, such as the white light, as the illumination light.

The IEE1 in the illumination light timing 75 is a timing at which the light source device 5 emits first special light as the illumination light. The IEE2 in the illumination light timing 75 is a timing at which the light source device 5 emits second special light different from the first special light as the illumination light.

The first special light is special light having a first characteristic (spectrum) suitable for the pick-up analysis (first analysis) described above, and is associated with the pick-up analysis. As an example, the first special light is the illumination light including narrow-band short-wavelength light and the white light, and is the illumination light that can generate the captured image in which a minute tone change is enhanced as compared with the illumination light of only the white light. It should be noted that the first special light is not limited to this, and can be, for example, various types of the illumination light having characteristics suitable for the pick-up analysis.

The second special light is the special light having a second characteristic suitable for the differentiation analysis (second analysis) described above, and is associated with the differentiation analysis. As an example, the second special light is the illumination light of narrow-band short-wavelength light, and is the illumination light capable of generating the captured image suitable for observing a blood vessel, a surface structure, or the like. It should be noted that the second special light is not limited to this, and can be, for example, various types of the illumination light having characteristics suitable for the differentiation analysis.

As shown in the illumination light timing 75, the light source device 5 repeatedly executes a predetermined irradiation operation in a period T. The irradiation operation is an operation of emitting the normal light and then emitting the special light (first special light or second special light).

An imaging timing 76 is a timing at which the imaging element 23 performs imaging (exposure) in accordance with the command from the control device 4. A vertical direction at the imaging timing 76 indicates a position of the pixel row 62 in the column direction Y (see Fig. 4). As described above, since the imaging element 23 in the present embodiment performs imaging of the rolling shutter system, the imaging timing 76 deviates for each pixel row 62. In the example shown in Fig. 7, the imaging element 23 performs imaging at a frame rate of 60 frames per second (fps).

As shown in the illumination light timing 75 and the imaging timing 76, the first period in which the light source device 5 continuously emits the normal light extends over a plurality of consecutive frames in imaging by the imaging element 23. In addition, the second period in which the light source device 5 continuously emits the special light extends over at least one frame in imaging by the imaging element 23. In the example shown in Fig. 7, the second period extends over the plurality of consecutive frames by the imaging element 23.

As described above, the light source device 5 repeats the operation of continuously emitting the normal light over the plurality of consecutive imaging operation frames, and then emitting the special light. Moreover, as described above, the control device 4 displays the live image (motion picture) on the display 7 based on the captured image obtained during the irradiation with the normal light, and performs the analysis based on the captured image obtained during the irradiation with the special light.

In the example shown in Fig. 8, since the wide-area observation is performed until the time t1, the determination unit 42e determines that the target area of imaging is "wide". Therefore, the control device 4 causes the analysis unit 42c to execute the pick-up analysis until the time t1, and sets the illumination light emitted by the light source device 5 to the first special light. As a result, for example, the analysis unit 42c can execute the pick-up analysis based on the imaging frame 76a within a period in which the irradiation with the first special light suitable for the pick-up analysis is performed.

In addition, since the detailed observation is performed after the time t1, the determination unit 42e determines that the target area of imaging is "narrow". Therefore, the control device 4 causes the analysis unit 42c to execute the differentiation analysis after the time t1, and the illumination light emitted by the light source device 5 is set to the second special light. As a result, for example, the analysis unit 42c can execute the differentiation analysis based on the imaging frames 76b and 76c within a period in which the irradiation with the second special light suitable for the differentiation analysis is performed.

As described above, the control device 4 also switches the characteristic of the illumination light emitted by the illumination lens 50, in addition to the analysis of which the result is displayed on the display 7 among the plurality of types of analysis, in accordance with the result of determining the size of the target area of the imaging inside the subject. As a result, while presenting the result of the analysis in accordance with the observation situation, the analysis in accordance with the observation situation can be performed with high accuracy by the captured image obtained by the illumination light having an appropriate characteristic.

In addition, the configuration has been described in which at the time t1 at which the determination result by the determination unit 42e is changed from "wide" to "narrow", the control device 4 switches the analysis of which the result is displayed on the display 7 among the plurality of types of analysis and the characteristic of the illumination light, but the present invention is not limited to such a configuration.

For example, after a state in which the determination result by the determination unit 42e is "wide", in a case in which a state in which the determination result by the determination unit 42e is "narrow" continues for a predetermined time (as an example, 1 second), the control device 4 may perform switching described above. That is, even in a case in which the determination result by the determination unit 42e is changed from "wide" to "narrow", in a case in which the determination result by the determination unit 42e returns to "wide" within the predetermined time, the control device 4 may not perform switching described above.

In addition, after the determination result by the determination unit 42e is changed from "wide" to "narrow" and switching described above is performed, in a case in which the determination result by the determination unit 42e returns to "wide", the control device 4 may display the result of the pick-up analysis on the display 7 and return to the state in which the irradiation with the first special light is performed by the light source device 5. As a result, it is possible to handle a case in which the user returns from the detailed observation to the wide-area observation.

### (Another Example of Imaging Element 23)

Although the configuration has been described in which the imaging element 23 having the rolling shutter system is used, a configuration may be adopted in which the imaging element 23 having the global shutter system is used.

### (Another Example of Determination Stage)

The configuration has been described in which the determination unit 42e determines the size of the target area of imaging in two stages of "wide" and "narrow", but the determination unit 42e may determine the size of the target area of imaging in three or more stages. In this case, the control device 4 may switch the analysis of which the result is displayed on the display 7 and the characteristic of the illumination light in three or more ways in accordance with the determination result in three or more stages by the determination unit 42e.

### (Another Example 1 of Determination Method)

In addition, the method has been described in which the determination unit 42e is provided in the captured image information generation unit 42a, and the size of the target area of imaging is determined based on the captured image information generated by the captured image information generation unit 42a, but the position at which the determination unit 42e is provided or the determination method by the determination unit 42e is not limited to this.

For example, in a case in which the angle of view of the objective lens 21 or the lens group 22 is variable, the determination unit 42e may acquire the information indicating the current angle of view of the objective lens 21 or the lens group 22 and determine the size of the target area of imaging based on the acquired information.

### (Another Example 2 of Determination Method)

In addition, in a case in which the distal end of the endoscope 1 is provided with a measurement instrument that measures the distance from the target of imaging (inner surface inside the subject) by the imaging element 23, the determination unit 42e may acquire the information indicating the measurement result by the measurement instrument and determine the size of the target area of imaging by using the acquired information. As an example, in a case in which the measured distance is equal to or larger than a threshold value, it is determined that the size of the target area of imaging is "wide", and in a case in which the measured distance is less than the threshold value, it is determined that the size of the target area of imaging is "narrow".

### (Another example 3 of Determination Method)

In addition, in the endoscope apparatus 100, in a case in which a control of correcting the intensity of the illumination light emitted by the light source device 5 is performed such that the brightness of the obtained captured image is brightness in a predetermined range, the determination unit 42e may acquire the information indicating a result of correction and determine the size of the target area of imaging by using the acquired information.

For example, in a case in which the user performs wide-area observation, the distal end of the endoscope 1 is far from the inner surface inside the subject, so that it is difficult for the illumination light to reach the inner surface inside the subject. Therefore, the intensity of the illumination light emitted by the light source device 5 is controlled to be relatively high such that the brightness of the captured image is maintained.

On the other hand, in a case in which the user performs the detailed observation, the distal end of the endoscope 1 is close to the inner surface of the subject, so that the illumination light easily reaches the inner surface of the subject. Therefore, the intensity of the illumination light emitted by the light source device 5 is controlled to be relatively low such that the brightness of the captured image is maintained.

Therefore, as an example, the determination unit 42e determines that the size of the target area of imaging is "wide" in a case in which the intensity of the illumination light emitted by the light source device 5 is equal to or larger than a threshold value, and determines that the size of the target area of imaging is "narrow" in a case in which the intensity of the illumination light emitted by the light source device 5 is less than the threshold value.

### (Another Example 4 of Determination Method)

In addition, the determination unit 42e may determine the size of the target area of imaging by using at least two determination methods in combination among the determination methods described above.

### (Specific Example of Analysis of Presence or Absence of Abnormal Site)

The analysis of the presence or absence of the abnormal site by the analysis unit 42c is, for example, the detection of a region-of-interest inside the subject into which the endoscope 1 is inserted. For example, the analysis unit 42c detects the region-of-interest inside the subject from the image indicated by the captured image information obtained by imaging during the irradiation with the special light. The region-of-interest is an abnormal site, such as a region that is likely to be a lesion, and is a region that is recommended for attention in the observation of the inside of the subject. The analysis image generation unit 42d generates image information for displaying a region-of-interest-enhanced image in which the region-of-interest detected by the analysis unit 42c is enhanced in the image indicated by the captured image information obtained by imaging during the irradiation with the special light. As a result, the region-of-interest-enhanced image is displayed on a sub-screen 72, and the operator of the endoscope 1 can easily recognize the region-of-interest inside the subject. Alternatively, the analysis image generation unit 42d may generate image information for displaying color difference-expanded image subjected to color difference expansion processing of expanding a color difference between an abnormal site (lesion site), which is the region-of-interest, and a normal site in the image indicated by the captured image information obtained by imaging during the irradiation with the special light. As a result, the color difference-expanded image is displayed on the sub-screen 72, and the operator of the endoscope 1 can easily distinguish between the abnormal site and the normal site inside the subject.

### (Specific Example of Analysis of Analyzing Abnormal Site)

The analysis of analyzing the abnormal site by the analysis unit 42c may include, for example, selection of a similar case image. For example, the analysis unit 42c selects a case image similar to the captured image information obtained by imaging during the irradiation with the special light by searching a database accessible to the endoscope apparatus 100. The analysis image generation unit 42d generates image information for displaying an image indicating a selection result by the analysis unit 42c. The selection result by the analysis unit 42c may be the case image itself selected by the analysis unit 42c, or may be information, such as diagnosis result, relating to the case image associated with the case image selected by the analysis unit 42c in the database. As a result, the selection result of the similar case image is displayed on the sub-screen 72, and the operator of the endoscope 1 can easily compare a state inside the subject under observation with the similar case.

Alternatively, the analysis of analyzing the abnormal site by the analysis unit 42c may include determination between a tumor and a non-tumor. For example, the analysis unit 42c determines whether or not a biological region reflected in the image indicated by the captured image information obtained by imaging during the irradiation with the special light is the tumor. The analysis image generation unit 42d generates image information for displaying an image indicating a determination result by the analysis unit 42c. The determination result by the analysis unit 42c may be information indicating whether or not the biological region reflected in the most recently captured image is the tumor, or may be information indicating the number of the biological regions determined to be the tumor since the start of the current examination. As a result, the determination result of the tumor and the non-tumor is displayed on the sub-screen 72, and it is possible to support the observation or the operation of the endoscope 1 by the operator of the endoscope 1.

Alternatively, the analysis of analyzing the abnormal site by the analysis unit 42c may include specifying a state of an organ. For example, the analysis unit 42c specifies the state of the organ reflected in the image indicated by the captured image information obtained by imaging during the irradiation with the special light. Examples of the state of the organ include the oxygen saturation for each region, the thickness, the density, pattern, and the uniformity of the blood vessel structure, the surface structure of the large intestine (for example, pit pattern structure), or the surface structure of the duodenum (for example, villous structure). The analysis image generation unit 42d generates image information for displaying an image indicating a specifying result by the analysis unit 42c. For example, the analysis image generation unit 42d generates an oxygen saturation image obtained by imaging the oxygen saturation for each specified region. As a result, the specifying result of the state of the organ is displayed on the sub-screen 72, and it is possible to support the observation or the operation of the endoscope 1 by the operator of the endoscope 1.

Alternatively, the analysis of analyzing the abnormal site by the analysis unit 42c may include generation of a planned separation line. For example, the analysis unit 42c decides the planned separation line (demarcation line) which is the line to be separated to remove the tumor in the biological region reflected in the image indicated by the captured image information obtained by imaging during the irradiation with the special light. The analysis image generation unit 42d generates image information for displaying an image to which the planned separation line decided by the analysis unit 42c is attached in the image indicated by the captured image information obtained by imaging during the irradiation with the special light. As a result, the image to which the planned separation line is attached is displayed on the sub-screen 72, and the operator of the endoscope 1 can easily recognize the planned separation line inside the subject.

### (Modification Examples of First Period, Second Period, and Period T)

Although the configuration has been described in which the length of each of the first period in which the normal light is emitted and the second period in which the special light is emitted is fixed in the repetition for each period T, but the length of each of the first period in which the normal light is emitted and the second period in which the special light is emitted may not be fixed (may be variable) in the repetition for each period T. For example, a ratio of the lengths of the first period and the second period in one period T may be 3:1, and a ratio of the lengths of the first period and the second period in the other period T may be 3:2.

In addition, although the case has been described in which the period T, which is the repetition period of the operation of emitting the normal light and the special light, is fixed, the period T may be variable. In addition, the configuration has been described in which the normal light is first emitted and then the special light is emitted in the period T, a configuration may be adopted in which the special light is first emitted and then the normal light is emitted in the period T.

In addition, the spectrum of the normal light may be fixed in the repetition for each period T or may be variable in the repetition for each period T. Similarly, the spectrum of the special light may be fixed in the repetition for each period T or may be variable in the repetition for each period T.

In addition, although the configuration has been described in which the second period in which the special light is emitted is immediately after the first period in which the normal light is emitted, a non-irradiation period in which the light source device 5 does not emit the illumination light may be present between the first period and the second period.

In addition, a configuration may be adopted in which narrow-band short-wavelength dimming light and white light are simultaneously emitted as the normal light or the special light described above. As a result, minute differences in color are enhanced and displayed, and the observation, such as inflammation observation or pick-up observation, is facilitated.

### (Another Embodiment of Endoscope System)

The endoscope apparatus 100 has been described as an example of the endoscope system according to the embodiment of the present invention, the endoscope system according to the embodiment of the present invention may be realized by a plurality of devices connected to each other via the network. For example, a configuration may be adopted in which at least a part of the processing by the control device 4 described above is executed by another device connected to the endoscope apparatus 100 via the network.

### (Control Program)

A control program, which is stored in the ROM of the control device 4, is stored in a program computer-readable non-transitory storage medium. Examples of such a "computer-readable storage medium" include an optical medium, such as a compact disc-ROM (CD-ROM), and a magnetic storage medium, such as a universal serial bus (USB) memory, or a memory card. In addition, such a program can also be provided by being downloaded via a network.

According to the present invention, it is possible to provide the endoscope system, the control method, and the control program that can present a highly accurate result of analysis in accordance with an observation situation.

### Explanation of References

1: endoscope
4: control device
5: light source device
6: input unit
7: display
10: insertion part
10A: flexible part
10B: bendable part
10C: distal end part
11: operating part
12: angle knob
13: universal cord
13A: connector portion
13B: connector portion
21: objective lens
22: lens group
23: imaging element
25: memory
26, 41: communication I/F
27: imaging driving unit
42: signal processing unit
42a: captured image information generation unit
42b: live image generation unit
42c: analysis unit
42d: analysis image generation unit
42e: determination unit
43: display controller
44: system control unit
45: recording medium
50: illumination lens
51: light source processor
52: light source unit
52a: V-LED
52b: B-LED
52c: G-LED
52d: R-LED
53: light guide
54: optical path coupling unit
60: imaging surface
61: pixel
62: pixel row
63: drive circuit
64: signal processing circuit
70: screen
71: main screen
72: sub-screen
75: illumination light timing
76: imaging timing
76a, 76b, 76c: imaging frame
100: endoscope apparatus
t1: time

## Claims

1. An endoscope system comprising:
a light source (5) that is able to switch between a plurality of types of illumination light having different characteristics to perform irradiation;
a display (7) that is able to switch between results of a plurality of types of analysis based on a captured image obtained by imaging using the illumination light to display the results; and
a processor (4) that switches analysis of which a result is displayed on the display among the plurality of types of analysis and the characteristic of the illumination light emitted by the light source in accordance with a result of determining a size of a target area of the imaging inside a subject, **characterized in that** the light source (5) repeats an operation of continuously emitting illumination light having a first characteristic in a first period over at least one consecutive imaging operation frame, and then emitting illumination light having a second characteristic different from the first characteristic in a second period over at least one imaging operation frame,
the plurality of types of analysis are performed based on the captured image obtained by the imaging in the second period, and
the processor (4) switches the characteristic of the illumination light in the second period in accordance with the result of determining the size of the target area.

2. The endoscope system according to claim 1,
wherein the plurality of types of analysis includes first analysis of determining presence or absence of an abnormal site based on the captured image and second analysis of analyzing the abnormal site based on the captured image, and
the processor displays a result of the first analysis in a case in which the size of the target area is a first size, and displays a result of the second analysis in a case in which the size of the target area is a second size narrower than the first size.

3. The endoscope system according to claim 1 or 2,
wherein the plurality of types of analysis includes first analysis and second analysis which are different from each other, and
the processor
displays a result of the first analysis and sets the characteristic of the illumination light to a characteristic associated with the first analysis, in a case in which the size of the target area is a first size, and
displays a result of the second analysis and sets the characteristic of the illumination light to a characteristic associated with the second analysis, in a case in which the size of the target area is a second size narrower than the first size.

4. The endoscope system according to any one of claims 1 to 3,
wherein the processor displays a motion picture based on the captured image obtained by the imaging in the first period on the display or a display different from the display.

5. The endoscope system according to any one of claims 1 to 4,
wherein the illumination light having the first characteristic is white light, and
the illumination light having the second characteristic is light for image-enhanced endoscopy.

6. The endoscope system according to any one of claims 1 to 5,
wherein a length of each of the first period and the second period is fixed in repetition of the operation or is variable in repetition of the operation.

7. The endoscope system according to any one of claims 1 to 6,
wherein spectra of the illumination light having the first characteristic and the illumination light having the second characteristic are fixed in repetition of the operation or is variable in repetition of the operation.

8. The endoscope system according to any one of claims 1 to 7,
wherein a non-irradiation period of the light source is present between the first period and the second period, or wherein the first period is a period longer than the second period.

9. The endoscope system according to any one of claims 1 to 8,
wherein, after a state in which the size of the target area is a first size, in a case in which a state in which the size of the target area is a second size narrower than the first size continues for a predetermined time or longer, the processor switches the analysis of which the result is displayed on the display among the plurality of types of analysis and the characteristic of the illumination light emitted by the light source.

10. The endoscope system according to any one of claims 1 to 9,
wherein the processor determines the size of the target area of the imaging by using the captured image, or by using a measurement result of a distance between a distal end of an endoscope including an imaging element that performs the imaging, and a target of the imaging.

11. The endoscope system according to any one of claims 1 to 10,
wherein the imaging is performed by an optical system of which an angle of view is variable, and
the processor determines the size of the target area of the imaging by using the angle of view of the optical system.

12. The endoscope system according to any one of claims 1 to 11,
wherein intensity of the illumination light emitted by the light source is corrected in accordance with a distance between a distal end of an endoscope including an imaging element that performs the imaging, and a target of the imaging, and
the processor determines the size of the target area of the imaging by using a result of correcting the intensity.

13. The endoscope system according to claim 2,
wherein analysis of the presence or absence of the abnormal site includes detection of a region-of-interest inside the subject into which an endoscope including an imaging element that performs the imaging is inserted, and/or wherein analysis of the abnormal site includes at least any analysis of a type of a lesion or an extent of the lesion at the abnormal site, selection of a similar case image, determination between a tumour and a non-tumour, specifying a state of an organ, or generation of a planned separation line.

14. The endoscope system according to any one of claims 1 to 13,
wherein the imaging is performed by an imaging element having a rolling shutter system, or wherein the imaging is performed by an imaging element having a global shutter system.

15. A control program of an endoscope system including a light source (5) that is able to switch between a plurality of types of illumination light having different characteristics to perform irradiation, and a display (7) that is able to switch between results of a plurality of types of analysis based on a captured image obtained by imaging using the illumination light to display the results, the program comprising instructions which, when the control program is executed on a computer, cause the endoscope system to execute a process comprising:
switching analysis of which a result is displayed on the display among the plurality of types of analysis and the characteristic of the illumination light emitted by the light source in accordance with a result of determining a size of a target area of the imaging inside a subject, **characterized in that** the light source (5) repeats an operation of continuously emitting illumination light having a first characteristic in a first period over at least one consecutive imaging operation frame, and then emitting illumination light having a second characteristic different from the first characteristic in a second period over at least one imaging operation frame,
the plurality of types of analysis are performed based on the captured image obtained by the imaging in the second period, and
a processor (4) switches the characteristic of the illumination light in the second period in accordance with the result of determining the size of the target area.

## Patentansprüche

1. Endoskopsystem, umfassend:
eine Lichtquelle (5), die in der Lage ist, zwischen mehreren Typen von Beleuchtungslicht mit unterschiedlichen Eigenschaften umzuschalten, um Bestrahlung durchzuführen;
eine Anzeige (7), die in der Lage ist, zwischen Ergebnissen von mehreren Typen von Analyse auf der Grundlage eines aufgenommenen Bildes, das durch Abbilden unter Verwendung des Beleuchtungslichts erhalten wird, umzuschalten, um die Ergebnisse anzuzeigen; und
einen Prozessor (4), der Analyse, deren Ergebnis auf der Anzeige angezeigt wird, unter den mehreren Typen von Analyse, und die Eigenschaft des von der Lichtquelle emittierten Beleuchtungslichts in Übereinstimmung mit einem Ergebnis des Bestimmens einer Größe eines Zielbereichs der Bildgebung innerhalb einer Untersuchungsperson umschaltet,
**dadurch gekennzeichnet, dass**
die Lichtquelle (5) einen Vorgang des kontinuierlichen Emittierens von Beleuchtungslicht mit einer ersten Eigenschaft in einer ersten Periode über mindestens einen aufeinanderfolgenden Bildgebungsbetriebsrahmen und des anschließenden Emittierens von Beleuchtungslicht mit einer zweiten Eigenschaft, die sich von der ersten Eigenschaft unterscheidet, in einer zweiten Periode über mindestens einen Bildgebungsbetriebsrahmen wiederholt,
die mehreren Typen von Analyse auf der Grundlage des aufgenommenen Bildes, das durch die Bildgebung in der zweiten Periode erhalten wird, durchgeführt werden, und
der Prozessor (4) die Eigenschaft des Beleuchtungslichts in der zweiten Periode in Übereinstimmung mit dem Ergebnis des Bestimmens der Größe des Zielbereichs umschaltet.

2. Endoskopsystem nach Anspruch 1,
wobei die mehreren Typen von Analyse erste Analyse des Bestimmens von Vorhandensein oder Nichtvorhandensein einer abnormalen Stelle auf der Grundlage des aufgenommenen Bildes und zweite Analyse des Analysierens der abnormalen Stelle auf der Grundlage des aufgenommenen Bildes enthält und der Prozessor ein Ergebnis der ersten Analyse in einem Fall, in dem die Größe des Zielbereichs eine erste Größe ist, anzeigt und ein Ergebnis der zweiten Analyse in einem Fall, in dem die Größe des Zielbereichs eine zweite Größe, die schmaler als die erste Größe ist, ist, anzeigt.

3. Endoskopsystem nach Anspruch 1 oder 2,
wobei die mehreren Typen von Analyse erste Analyse und zweite Analyse, die sich voneinander unterscheiden, enthält, und
der Prozessor
ein Ergebnis der ersten Analyse anzeigt und die Eigenschaft des Beleuchtungslichts auf eine Eigenschaft, die der ersten Analyse zugeordnet ist, in einem Fall, in dem die Größe des Zielbereichs eine erste Größe ist, einstellt, und
ein Ergebnis der zweiten Analyse anzeigt und die Eigenschaft des Beleuchtungslichts auf eine Eigenschaft, die der zweiten Analyse zugeordnet ist, in einem Fall, in dem die Größe des Zielbereichs eine zweite Größe, die schmaler als die erste Größe ist, ist, einstellt.

4. Endoskopsystem nach einem der Ansprüche 1 bis 3,
wobei der Prozessor einen Film auf der Grundlage des aufgenommenen Bildes, das durch die Bildgebung in der ersten Periode erhalten wird, auf der Anzeige oder einer von der Anzeige verschiedenen Anzeige anzeigt.

5. Endoskopsystem nach einem der Ansprüche 1 bis 4,
wobei das Beleuchtungslicht mit der ersten Eigenschaft Weißlicht ist, und
das Beleuchtungslicht mit der zweiten Eigenschaft Licht für bildverstärkte Endoskopie ist.

6. Endoskopsystem nach einem der Ansprüche 1 bis 5,
wobei eine Länge jeweils der ersten Periode und der zweiten Periode bei Wiederholung des Vorgangs fixiert ist oder bei Wiederholung des Vorgangs variabel ist.

7. Endoskopsystem nach einem der Ansprüche 1 bis 6,
wobei Spektren des Beleuchtungslichts mit der ersten Eigenschaft und des Beleuchtungslichts mit der zweiten Eigenschaft bei Wiederholung des Vorgangs fixiert sind oder bei Wiederholung des Vorgangs variabel sind.

8. Endoskopsystem nach einem der Ansprüche 1 bis 7,
wobei eine Nichtbestrahlungsperiode der Lichtquelle zwischen der ersten Periode und der zweiten Periode vorhanden ist oder wobei die erste Periode eine Periode, die länger als die zweite Periode ist, ist.

9. Endoskopsystem nach einem der Ansprüche 1 bis 8,
wobei, nach einem Zustand, in dem die Größe des Zielbereichs eine erste Größe ist, in einem Fall, in dem ein Zustand, in dem die Größe des Zielbereichs eine zweite Größe, die schmaler als die erste Größe ist, ist, für eine vorbestimmte Zeit oder länger andauert, der Prozessor die Analyse, deren Ergebnis auf der Anzeige angezeigt wird, unter den mehreren Typen von Analyse und die Eigenschaft des von der Lichtquelle emittierten Beleuchtungslichts umschaltet.

10. Endoskopsystem nach einem der Ansprüche 1 bis 9,
wobei der Prozessor die Größe des Zielbereichs der Bildgebung durch Verwenden des aufgenommenen Bildes oder durch Verwenden eines Messergebnisses eines Abstands zwischen einem distalen Ende eines Endoskops, das ein Bildgebungselement enthält, das die Bildgebung durchführt, und einem Ziel der Bildgebung bestimmt.

11. Endoskopsystem nach einem der Ansprüche 1 bis 10,
wobei die Bildgebung durch ein optisches System durchgeführt wird, dessen Blickwinkel variabel ist, und
der Prozessor die Größe des Zielbereichs der Bildgebung durch Verwenden des Blickwinkels des optischen Systems bestimmt.

12. Endoskopsystem nach einem der Ansprüche 1 bis 11,
wobei Intensität des von der Lichtquelle emittierten Beleuchtungslichts in Übereinstimmung mit einem Abstand zwischen einem distalen Ende eines Endoskops, das ein Bildgebungselement enthält, das die Bildgebung durchführt, und einem Ziel der Bildgebung korrigiert wird, und
der Prozessor die Größe des Zielbereichs der Bildgebung durch Verwenden eines Ergebnisses des Korrigierens der Intensität bestimmt.

13. Endoskopsystem nach Anspruch 2,
wobei Analyse des Vorhandenseins oder Nichtvorhandenseins der abnormen Stelle Detektion eines Bereichs von Interesse innerhalb der Untersuchungsperson, in die ein Endoskop, das ein Bildgebungselement enthält, das die Bildgebung durchführt, eingeführt wird, enthält, und/oder wobei Analyse der abnormen Stelle mindestens eine Analyse eines Typs einer Läsion oder eines Ausmaßes der Läsion an der abnormen Stelle, Auswahl eines ähnlichen Fallbildes, Bestimmung zwischen einem Tumor und einem Nichttumor, Spezifizieren eines Zustands eines Organs oder Erzeugung einer geplanten Trennlinie enthält.

14. Endoskopsystem nach einem der Ansprüche 1 bis 13,
wobei die Bildgebung durch ein Bildgebungselement, das ein Rollingshutter-System aufweist, durchgeführt wird, oder wobei die Bildgebung durch ein Bildgebungselement, das ein Globalshutter-System aufweist, durchgeführt wird.

15. Steuerprogramm eines Endoskopsystems, das eine Lichtquelle (5), die in der Lage ist, zwischen mehreren Typen von Beleuchtungslicht mit unterschiedlichen Eigenschaften umzuschalten, um Bestrahlung durchzuführen, und eine Anzeige (7), die in der Lage ist, zwischen Ergebnissen von mehreren Typen von Analyse auf der Grundlage eines aufgenommenen Bildes, das durch Bildgebung unter Verwendung des Beleuchtungslichts erhalten wird, umzuschalten, um die Ergebnisse anzuzeigen, enthält, wobei das Programm Anweisungen umfasst, die, wenn das Steuerprogramm auf einem Computer ausgeführt wird, das Endoskopsystem veranlassen, einen Prozess auszuführen, der umfasst:
Umschalten von Analyse, deren Ergebnis auf der Anzeige angezeigt wird, unter den mehreren Typen von Analyse und der Eigenschaft des von der Lichtquelle emittierten Beleuchtungslichts in Übereinstimmung mit einem Ergebnis des Bestimmens einer Größe eines Zielbereichs der Bildgebung innerhalb einer Untersuchungsperson,
**dadurch gekennzeichnet, dass**
die Lichtquelle (5) einen Vorgang des kontinuierlichen Emittierens von Beleuchtungslicht mit einer ersten Eigenschaft in einer ersten Periode über mindestens einen aufeinanderfolgenden Bildgebungsbetriebsrahmen und des anschließenden Emittierens von Beleuchtungslicht mit einer zweiten Eigenschaft, die sich von der ersten Eigenschaft unterscheidet, in einer zweiten Periode über mindestens einen Bildgebungsbetriebsrahmen wiederholt,
die mehreren Typen von Analyse auf der Grundlage des aufgenommenen Bildes, das durch die Bildgebung in der zweiten Periode erhalten wird, durchgeführt werden, und
ein Prozessor (4) die Eigenschaft des Beleuchtungslichts in der zweiten Periode in Übereinstimmung mit dem Ergebnis des Bestimmens der Größe des Zielbereichs umschaltet.

## Revendications

1. Système d'endoscope comprenant :
une source de lumière (5) qui est capable de commuter entre une pluralité de types de lumière d'éclairage ayant des caractéristiques différentes pour effectuer une irradiation ;
un affichage (7) qui est capable de commuter entre des résultats d'une pluralité de types d'analyse sur la base d'une image capturée obtenue par imagerie en utilisant la lumière d'éclairage pour afficher les résultats ; et
un processeur (4) qui commute une analyse dont un résultat est affiché sur l'affichage parmi la pluralité de types d'analyse et la caractéristique de la lumière d'éclairage émise par la source de lumière en fonction d'un résultat de détermination d'une taille d'une zone cible de l'imagerie à l'intérieur d'un sujet,
**caractérisé en ce que**
la source de lumière (5) répète une opération consistant à émettre de manière continue une lumière d'éclairage ayant une première caractéristique pendant une première période sur au moins une trame d'opération d'imagerie consécutive, puis à émettre une lumière d'éclairage ayant une deuxième caractéristique différente de la première caractéristique pendant une deuxième période sur au moins une trame d'opération d'imagerie,
la pluralité de types d'analyse sont effectuées sur la base de l'image capturée obtenue par l'imagerie pendant la deuxième période, et
le processeur (4) commute la caractéristique de la lumière d'éclairage pendant la deuxième période en fonction du résultat de détermination de la taille de la zone cible.

2. Système d'endoscope selon la revendication 1,
dans lequel la pluralité de types d'analyse inclut une première analyse consistant à déterminer la présence ou l'absence d'un site anormal sur la base de l'image capturée et une deuxième analyse consistant à analyser le site anormal sur la base de l'image capturée, et le processeur affiche un résultat de la première analyse dans un cas où la taille de la zone cible est une première taille, et affiche un résultat de la deuxième analyse dans un cas où la taille de la zone cible est une deuxième taille plus étroite que la première taille.

3. Système d'endoscope selon la revendication 1 ou la revendication 2,
dans lequel la pluralité de types d'analyse inclut une première analyse et une deuxième analyse qui sont différentes l'une de l'autre, et
le processeur
affiche un résultat de la première analyse et règle la caractéristique de la lumière d'éclairage sur une caractéristique associée à la première analyse, dans un cas où la taille de la zone cible est une première taille, et
affiche un résultat de la deuxième analyse et règle la caractéristique de la lumière d'éclairage sur une caractéristique associée à la deuxième analyse, dans un cas où la taille de la zone cible est une deuxième taille plus étroite que la première taille.

4. Système d'endoscope selon l'une quelconque des revendications 1 à 3,
dans lequel le processeur affiche une image animée sur la base de l'image capturée obtenue par l'imagerie pendant la première période sur l'affichage ou sur un affichage différent de l'affichage.

5. Système d'endoscope selon l'une quelconque des revendications 1 à 4,
dans lequel la lumière d'éclairage ayant la première caractéristique est une lumière blanche, et
la lumière d'éclairage ayant la deuxième caractéristique est une lumière pour une endoscopie à image améliorée.

6. Système d'endoscope selon l'une quelconque des revendications 1 à 5,
dans lequel une durée de chacune de la première période et de la deuxième période est fixe lors de la répétition de l'opération ou est variable lors de la répétition de l'opération.

7. Système d'endoscope selon l'une quelconque des revendications 1 à 6,
dans lequel des spectres de la lumière d'éclairage ayant la première caractéristique et de la lumière d'éclairage ayant la deuxième caractéristique sont fixes lors de la répétition de l'opération ou sont variables lors de la répétition de l'opération.

8. Système d'endoscope selon l'une quelconque des revendications 1 à 7,
dans lequel une période de non-irradiation de la source de lumière est présente entre la première période et la deuxième période, ou dans lequel la première période est une période plus longue que la deuxième période.

9. Système d'endoscope selon l'une quelconque des revendications 1 à 8,
dans lequel, après un état dans lequel la taille de la zone cible est une première taille, dans un cas où un état dans lequel la taille de la zone cible est une deuxième taille plus étroite que la première taille se poursuit pendant une durée prédéterminée ou plus, le processeur commute l'analyse dont le résultat est affiché sur l'affichage parmi la pluralité de types d'analyse et la caractéristique de la lumière d'éclairage émise par la source de lumière.

10. Système d'endoscope selon l'une quelconque des revendications 1 à 9,
dans lequel le processeur détermine la taille de la zone cible de l'imagerie en utilisant l'image capturée, ou en utilisant un résultat de mesure d'une distance entre une extrémité distale d'un endoscope incluant un élément d'imagerie qui effectue l'imagerie, et une cible de l'imagerie.

11. Système d'endoscope selon l'une quelconque des revendications 1 à 10,
dans lequel l'imagerie est effectuée par un système optique dont un angle de vue est variable, et
le processeur détermine la taille de la zone cible de l'imagerie en utilisant l'angle de vue du système optique.

12. Système d'endoscope selon l'une quelconque des revendications 1 à 11,
dans lequel l'intensité de la lumière d'éclairage émise par la source de lumière est corrigée en fonction d'une distance entre une extrémité distale d'un endoscope incluant un élément d'imagerie qui effectue l'imagerie, et une cible de l'imagerie, et le processeur détermine la taille de la zone cible de l'imagerie en utilisant un résultat de la correction de l'intensité.

13. Système d'endoscope selon la revendication 2,
dans lequel l'analyse de la présence ou de l'absence du site anormal inclut la détection d'une région d'intérêt à l'intérieur du sujet dans lequel est inséré un endoscope incluant un élément d'imagerie qui effectue l'imagerie, et/ou dans lequel l'analyse du site anormal inclut au moins l'une quelconque parmi une analyse d'un type d'une lésion ou d'une étendue de la lésion au niveau du site anormal, une sélection d'une image de cas similaire, une détermination entre une tumeur et une non-tumeur, une spécification d'un état d'un organe, ou une génération d'une ligne de séparation planifiée.

14. Système d'endoscope selon l'une quelconque des revendications 1 à 13,
dans lequel l'imagerie est effectuée par un élément d'imagerie ayant un système d'obturateur roulant, ou dans lequel l'imagerie est effectuée par un élément d'imagerie ayant un système d'obturateur global.

15. Programme de contrôle d'un système d'endoscope incluant une source de lumière (5) qui est capable de commuter entre une pluralité de types de lumière d'éclairage ayant des caractéristiques différentes pour effectuer une irradiation, et un affichage (7) qui est capable de commuter entre des résultats d'une pluralité de types d'analyse sur la base d'une image capturée obtenue par imagerie en utilisant la lumière d'éclairage pour afficher les résultats, le programme comprenant des instructions qui, lorsque le programme de contrôle est exécuté sur un ordinateur, amènent le système d'endoscope à exécuter un processus comprenant :
commuter une analyse dont un résultat est affiché sur l'affichage parmi la pluralité de types d'analyse et la caractéristique de la lumière d'éclairage émise par la source de lumière en fonction d'un résultat de détermination d'une taille d'une zone cible de l'imagerie à l'intérieur d'un sujet,
**caractérisé en ce que**
la source de lumière (5) répète une opération consistant à émettre de manière continue une lumière d'éclairage ayant une première caractéristique pendant une première période sur au moins une trame d'opération d'imagerie consécutive, puis à émettre une lumière d'éclairage ayant une deuxième caractéristique différente de la première caractéristique pendant une deuxième période sur au moins une trame d'opération d'imagerie,
la pluralité de types d'analyse sont effectuées sur la base de l'image capturée obtenue par l'imagerie pendant la deuxième période, et
un processeur (4) commute la caractéristique de la lumière d'éclairage pendant la deuxième période en fonction du résultat de détermination de la taille de la zone cible.
